# EUROPEAN PATENT APPLICATION

(11) **EP 4 560 640 A1**
(43) Date of publication of application: **28.05.2025**
(21) Application number: 24846666.6
(22) Date of filing: 24.09.2024
(51) Int. Cl.: G16H 10/00, G16H 50/20

(54) **DIAGNOSIS AND PRESCRIPTION ASSISTANCE SYSTEM**

(30) Priority: 04.10.2023 JP 2023172400
(71) Applicant: Iryou Jyouhou Gijyutu Kenkyusho Corporation, Yame-gun, Fukuoka 834-0115 (JP)
(72) Inventor: SHINKICHI, Himeno, Fukuoka 8340102 (JP)
(74) Representative: dompatent von Kreisler Selting Werner - Partnerschaft von Patent- und Rechtsanwälten mbB
(86) International application number: PCT/JP2024/033809
(87) International publication number: WO 2025/074890

(57) **Abstract**

To support obtaining a confirmed diagnosis by causing a large language model to additionally learn patient records of a large number of diagnosed cases, causing the additionally trained large language model to propose an efficient test plan for confirming a diagnosis from symptom findings obtained from an undiagnosed patient visiting a hospital, and integrating obtained test results.

An electronic health record system using a large language model in combination includes: a "possible diagnosis name, additional symptom, physical finding, and test answering means" that answers at least one of a list of possible diagnosis names and probabilities of the diagnosis names, and a recommended additional symptom, physical finding, and test; and a sequential diagnosis improvement means for repeating a "symptom, physical finding, and test result data provision means" and the "possible diagnosis name, additional symptom, physical finding, and test answering means" until the list of possible diagnosis names and probabilities of the diagnosis names has sufficient accuracy for confirmed diagnosis.

## Description

### Technical Field

The present invention relates to a diagnosis and prescription support system that prepares a test plan from patient's symptoms and physical findings using a large language model, supports disease name diagnosis of a disease from the results, and further supports preparation of an appropriate treatment plan.

### Background Art

In medical settings or the like, a computer system (electronic health record) for efficiently recording medical instructions (orders) or records has been widely used.

In addition, in recent years, the progress of artificial intelligence (AI) has been remarkable. In particular, large language models (LLMs) or generative AI have attracted attention that enable a question and a response in natural language by learning a large amount of documents and data.

Background art documents related to this application include the following.

### Citation List

### Patent Literature

Patent Literature 1: JP 2023-523644 A
Patent Literature 2**:** JP 2023-73095 A
Patent Literature 3: JP 5484317 B2
Patent Literature 4: JP 7313757 B2
Patent Literature 5: JP 6792750 B2
Patent Literature 6: JP 7284970 B2
Patent Literature 7: JP 7145367 B2

### Summary of Invention

### Technical Problem

In implementation of medical care, test plans are arranged on the basis of patient's symptoms and physical examination findings, a treatment plan is made according to the test results, and treatment is performed. Since medical practitioners of various occupations such as doctors and nurses are involved, it goes without saying that electronic health records are useful for efficient medical activities.

It is necessary to examine a patient, narrow down a disease name to some extent from symptoms, physical findings, and the like, determine the disease name by performing a test, and prepare a treatment plan on the basis of the disease name.

Arrangement of medical instructions (orders) such as tests and treatments, and the like are manually performed by doctors. This requires a vast amount of work, resulting in long working hours of doctors. In addition, there is a problem in that an erroneous diagnosis or unnecessary test occurs or a therapeutic effect varies due to a difference in knowledge or experience level for each doctor.

The present invention has been made to solve such conventional problems, and an object of the present invention is**:** to support obtaining a confirmed diagnosis by causing a large language model to additionally learn patient records of a large number of diagnosed cases (additionally trained large language model), causing the additionally trained large language model to propose an efficient test plan for confirming a diagnosis from symptom findings obtained from an undiagnosed patient visiting a hospital, and integrating obtained test results; and to provide a diagnosis and prescription support system that proposed an appropriate treatment plan on the basis of the confirmed diagnosis.

### Solution to Problem

As a means for achieving the object, a diagnosis and prescription support system according to claim 1 includes:
in an electronic health record system using a large language model in combination,
(1) an additional learning data provision means for diagnosis for performing additional learning by an attention mechanism for obtaining a correlation between an individual symptom, physical finding, test finding, or the like and a disease name of a diagnosed case which is a teacher label, and a correlation between an individual symptom, physical finding, test finding, and the like, as an attention weight of each correlation by using diagnosis name, symptom, physical finding, and test result data of the diagnosed case extracted from an electronic health record, to obtain an additionally trained large language model (a means for creating additionally trained large language model using diagnosed case data);
(2) a "symptom, physical finding, and test result data provision means" that provides symptom, physical finding, and test result data in an electronic health record obtained for an undiagnosed case to the additionally trained large language model;
(3) a "possible diagnosis name, additional symptom, physical finding, and test answering means" that answers, by the additionally trained large language model, at least one of a list of possible diagnosis names and probabilities of the diagnosis names, and a recommended additional symptom, physical finding, and test by calculating probabilities of respective disease names at an intermediate elapsed time point at which the symptom, finding, test result, and the like are obtained, using attention weights of symptom, physical finding, and test result data groups obtained by the symptom, physical finding, and test result data provision means, and a context vector obtained by integrating the attention weights; and
(4) a sequential diagnosis improvement means for repeating the "symptom, physical finding, and test result data provision means" and the "possible diagnosis name, additional symptom, physical finding, and test answering means".

A diagnosis and prescription support system according to claim 2 is the diagnosis and prescription support system according to claim 1, further including a recommended test order creation means for creating a test order recommended for an electronic health record by the "possible diagnosis name, additional symptom, physical finding, and test answering means".

A diagnosis and prescription support system according to claim 3 is the diagnosis and prescription support system according to claim 1 or 2, in which the additional learning provision means for diagnosis includes treatment items such as treatment including administration medicine, injection, rehabilitation, and surgery as well as "symptom, physical finding, and test finding" for proceeding with diagnosis of a diagnosed case.

A diagnosis and prescription support system according to claim 4 is the diagnosis and prescription support system according to claim 1 or 2, in which the additionally trained large language model includes a treatment plan proposal means for proposing a treatment plan of treatment including administration medicine, injection, rehabilitation, and surgery for a confirmed diagnosis obtained by the sequential diagnosis improvement means.

A diagnosis and prescription support system according to claim 5 is the diagnosis and prescription support system according to claim 4, further including a recommended treatment order creation means for creating, for an electronic health record, an individual treatment order such as treatment including administration medicine, injection, rehabilitation, and surgery proposed by the treatment plan proposal means.

A diagnosis and prescription support system according to claim 6 is the diagnosis and prescription support system according to claim 4, further including a progress log creation means for recording progress logs of the sequential diagnosis improvement means and the treatment plan proposal means.

### Advantageous Effects of Invention

The diagnosis and prescription support system according to claim 1 includes the additional learning data provision means for diagnosis, and performs additional learning by the attention mechanism for obtaining a correlation between an individual symptom, physical finding, test finding, or the like and a disease name of a diagnosed case which is a teacher label, and a correlation between an individual symptom, physical finding, test finding, and the like, as an attention weight of each correlation by using diagnosis name, symptom, physical finding, and test result data of the diagnosed case extracted from an electronic health record, to obtain an additionally trained large language model (a means for creating additionally trained large language model using diagnosed case data).

The "symptom, physical finding, and test result data provision means" is included, and provides symptom, physical finding, and test result data in an electronic health record obtained for an undiagnosed case to the additionally trained large language model.

The "possible diagnosis name, additional symptom, physical finding, and test answering means" is included, and answers at least one of a list of possible diagnosis names and probabilities of the diagnosis names, and a recommended additional symptom, physical finding, and test by calculating probabilities of respective disease names at an intermediate elapsed time point at which the symptom, finding, test result, and the like are obtained, using attention weights of symptom, physical finding, and test result data groups obtained by the symptom, physical finding, and test result data provision means, and a context vector obtained by integrating the attention weights.

The sequential diagnosis improvement means is included, and repeats the "symptom, physical finding, and test result data provision means" and the "possible diagnosis name, additional symptom, physical finding, and test answering means" until the list of possible diagnosis names and probabilities of the diagnosis names has sufficient accuracy for confirmed diagnosis.

The diagnosis and prescription support system according to claim 2 includes the recommended test order creation means, and creates a test order recommended for an electronic health record.

The diagnosis and prescription support system according to claim 3 includes treatment items such as treatment including administration medicine, injection, rehabilitation, and surgery as well as "symptom, physical finding, and test finding" for proceeding with diagnosis of a diagnosed case.

The diagnosis and prescription support system according to claim 4 proposes a treatment plan of treatment including administration medicine, injection, rehabilitation, and surgery for a confirmed diagnosis obtained by the sequential diagnosis improvement means.

The diagnosis and prescription support system according to claim 5 creates, for an electronic health record, an individual treatment order such as treatment including administration medicine, injection, rehabilitation, and surgery proposed by the treatment plan proposal means.

The diagnosis and prescription support system according to claim 6 includes the progress log creation means, and records progress logs of the sequential diagnosis improvement means and the treatment plan proposal means.

### Brief Description of Drawings

Fig. 1 is a hardware configuration diagram of the present invention.
Fig. 2 is an explanatory diagram for performing additional training of a large language model (LLM) using a large amount of diagnosed case data to create an additionally trained large language model.
Fig. 3 illustrates a relationship between basic learning operation and additional learning by a large language model.
Fig. 4 is an explanatory diagram of additional learning in the present invention.
Fig. 5 is an explanatory diagram for creating a diagnosis and treatment plan while receiving a proposal using an additionally trained large language model.
Fig. 6 illustrates an example of a diagnosis and treatment planning process.

### Description of Embodiments

A diagnosis and prescription support system according to the present invention includes a server device, a database, and a terminal.

The server device is a known computer device, and includes an arithmetic device, a main storage device, an auxiliary storage device, an input device, an output device, and a communication device.

The arithmetic device, the main storage device, the auxiliary storage device, the input device, the output device, and the communication device are connected to each other via a bus interface.

The arithmetic device includes a known processor capable of executing a command set.

The main storage device includes a volatile memory such as a RAM capable of temporarily storing the command set.

The auxiliary storage device includes a non-volatile data storage capable of recording an OS and a program.

The data storage may be, for example, an HDD or an SSD.

The input device is, for example, a keyboard.

The output device is, for example, a display such as an LCD.

The communication device includes a network interface connectable to a network.

The server device includes means such as a means for creating additionally trained large language model using diagnosed case data, an additional learning data provision means for diagnosis, a symptom, physical finding, and test result data provision means, a possible diagnosis name, additional symptom, physical finding, and test answering means, a sequential diagnosis improvement means, a recommended test order creation means, a treatment plan proposal means, a recommended treatment order creation means, and a progress log creation means.

The processor of the server device exerts a functional effect of the means.

The database according to the present invention may be configured by the auxiliary storage device of the server device, or may be configured by another auxiliary storage device independent of the server device.

The database stores information handled by the diagnosis and prescription support system.

The terminal according to the present invention has a hardware configuration of a known computer similarly to the server device.

The server device, the database, and the terminal according to the present invention can communicate via a network.

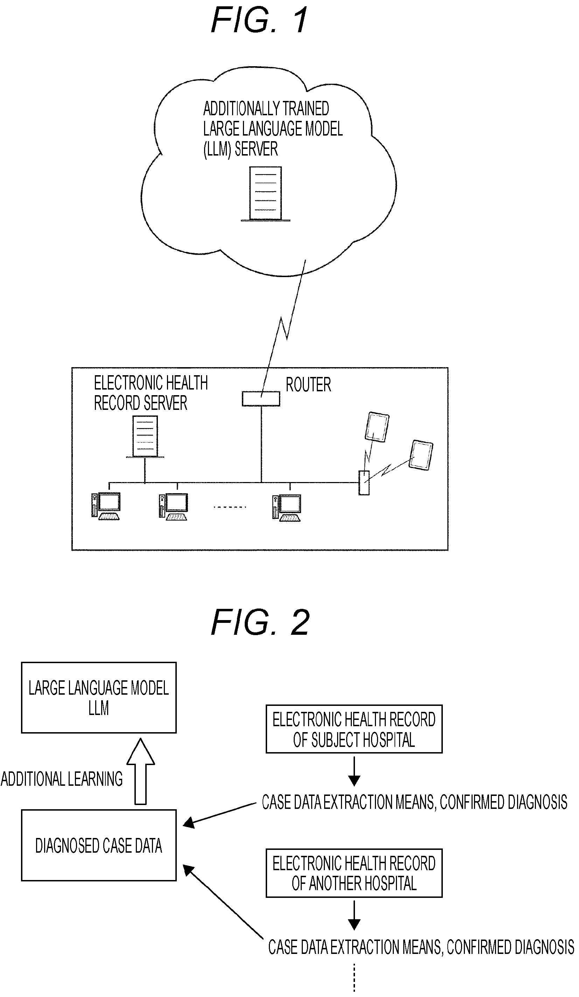

Fig. 1 is an example of a hardware configuration diagram of the present invention.

Because an additionally trained large language model requires huge data and a large amount of computational resources, the additionally trained large language model is usually on a cloud, and is connected to a local area network (LAN) in a medical institution via a router from an Internet line.

There is a server for electronic health records in the medical institution.

A staff member such as a doctor and a nurse uses the electronic health records and the large language model by using the terminal connected to the LAN.

Note that a part or all of the electronic health record system can be constructed on a cloud.

Also, a part or all of a lightweight, additionally trained large language model can be installed in the medical institution (edge computing). If this is possible, the response to an inquiry can be speeded up, and the risk of information leakage can be further reduced.

Fig. 2 is an explanatory diagram for performing additional training of a large language model (LLM) using a large amount of diagnosed case data to create an additionally trained large language model.

Case data such as symptoms, physical findings, and test results are extracted from the electronic health record (a case data extraction means), "diagnosed case data" is created together with a confirmed diagnosis that has already been obtained, and used for additional learning by a large language model (a means for creating additionally trained large language model using diagnosed case data).

In a large language model, a fictitious reaction called "hallucinations" may be returned, which is problematic. A "hallucination" occurs with respect to an inquiry about a content that is not in the learned data. This problem can be prevented by limiting the scope of a search to the large amount of diagnosed case data used for training.

If it is possible, improvement in accuracy can be expected when diagnosed case data of other hospitals as well as the subject hospital can be used.

Even if there is the same symptom of "chest pain", "angina" or "myocardial infarction" is suspected in a hospital specializing in circulatory organs, but there is a high possibility of "rib fracture" in a hospital specializing in orthopedic surgery. For this reason, it would also be useful to group diagnosed case data for each hospital as appropriate.

The large language model is currently being developed rapidly, and many models including ChatGPT (registered trademark of OpenAI), Bard, LaMDA (registered trademark of Google), and LLaMA (registered trademark of Meta Platforms) have been developed.

The large language model learns a large amount of data to configure a general-purpose language model (substrate trained model). However, knowledge in a specific field is not necessarily deep. Therefore, the large language model is caused to intensively additionally learn knowledge in a specific field to configure a dedicated model (the present invention is part of this).

In addition, it is known that the large language model of the general-purpose substrate trained model is weak against numerical calculation and logical operation. To compensate for this weakness, there is a dedicated module, such as Wolfram Alpha (Non Patent Literature 1), which responds to prompt questions from the LLM and return responses.

Meanwhile, it is conceivable to ask a WEB search engine a question and acquire a response.

As one of methods for solving such a problem, Function Calling has been proposed. This is to use another search engine, another database, or the function of a large language model specialized for a specific purpose via an application programming interface (API) from the LLM. Moreover, when the dedicated large language model or the like can be used in parallel, the responding time can be expected to decrease.

Furthermore, by enabling information reference from the electronic health record system and writing to the electronic health record system as will be described later, the electronic health record system can be directly controlled from the large language model using the Function Calling.

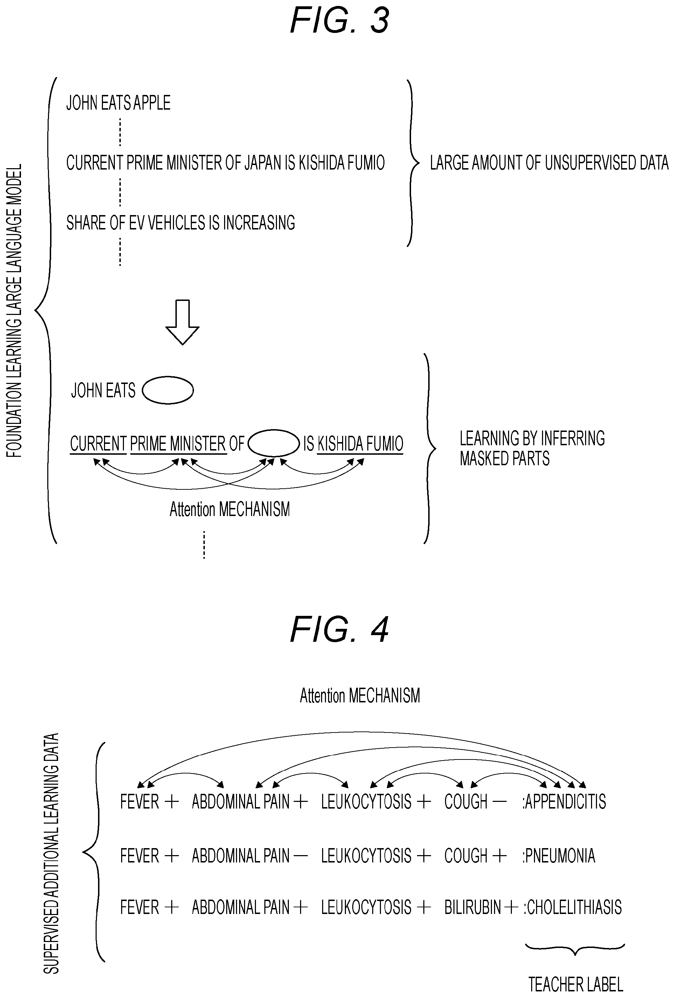

Fig. 3 illustrates a relationship between basic learning operation and additional learning by a large language model.

A general-purpose large language model collects as much document data as possible from the web or the like.

Any document element (for example, a word) included in the collected document data is masked, and learning to predict the masked document element from the remaining other document elements is performed. In the course of this learning, the relationship between the document elements is grasped by a method called the attention mechanism.

For a document generation task, a document element that is highly likely to come next is predicted on the basis of a document element having high relevance and a document element group generated so far, and a document element having the highest probability is adopted.

These are achieved by integrating the vector representation of each document element multiplied by the attention weight for each of the partially created document element columns and calculating the context vector. When a document element is added, a procedure of predicting a next document element is further repeated, so that a long sentence document can be generated.

Conventional learning models have required a large number of documents (corpuses) to which teacher labels are manually assigned.

The size of the corpus has been limited because the costly labeling has been a bottleneck. Large language models mask a portion of a document and predict the masked document elements on the basis of the rest of the document. Since the masked document elements are the ground truth (teacher labels), a large number of corpuses have been made available from unsupervised documents without human intervention.

In a conventional recurrent neural network (RNN) or the like, only document elements in the vicinity of document elements to be output are used, and document elements at distant positions are "forgotten". Therefore, it has been known that accuracy rapidly drops when the number of words exceeds several tens of words even if ingenuity is made. In LLMs, using the attention mechanism, remotely located document elements can also be used for prediction, and long sentences can also be generated.

Fig. 4 is an explanatory diagram of additional learning in the present invention.

Since the large language model collects and learns a large number of documents in all fields, general document understanding and generation are performed without problems. However, accuracy in a specific field is not necessarily high. For this reason, the large language model can be caused to additionally learn a document related to the specific field, making it an additionally trained large language model suitable for the specific field as a whole.

In the reinforcement learning of the present invention, symptoms, physical findings, and test results of a case for which a diagnosis has been confirmed are used as supervised additional learning data with the confirmed diagnosis as a teacher label.

At this time, information extraction using tag information from an electronic health record according to Patent Literature 7: JP 7145367 B2 may also be useful.

Note that the additional learning includes "transition learning" in which only the output layer is added without changing the parameter of the substrate learning model, and fine-tuning in which the parameter of the substrate learning model is relearned using the additional learning data. Although both are possible, fine tuning is more desirable in the present invention.

By learning using the attention mechanism, a relationship between individual symptoms, physical findings, test findings, and the like and a disease name which is a teacher label is obtained.

Furthermore, relationships between individual symptoms, physical findings, and test findings will also be learned.

The probability for each disease name can be calculated at an intermediate elapsed time point at which some symptoms, findings, test results, and the like are obtained using attention weights of each of the originally obtained symptoms and the physical findings groups, and a context vector obtained by integrating the attention weights.

That is, in a normal large language model, the document element having the highest probability is added one after another at the end of the already generated document element sequence, but in the present invention, the probability of each disease name at a certain time point is calculated using the attention mechanism from the symptoms, physical findings, test findings, and the like observed in a patient at the certain time point. This corresponds to the posterior probability of each possible disease name after the symptoms, physical findings, and test findings are partially obtained.

In Fig. 4, "fever", "abdominal pain", and the like are aligned for convenience of explanation, but since individual document element vectors and attention weights of the document element vectors are calculated by the attention mechanism, the appearance order of individual symptoms, physical findings, and test findings is optional.

It is desirable but not essential that all items of data are present. Even if the data is incomplete, since certain information is included, it is possible to contribute to output accuracy.

In addition, notation may be varied. For example, "+" in Fig. 4 may be "present", "positive", or the like. Furthermore, LLMs are good at translation, and it is also possible to mix multiple languages such as Japanese, English, French, and German.

The symptoms, physical findings, and test findings are sufficient to proceed with diagnosis. However, in order to further receive a proposal for a treatment plan from an additionally trained large language model, it is necessary to include treatment elements such as administration drugs, injections, rehabilitation, and surgery in the additional learning data (an additional learning provision means for diagnosis and treatment).

Since the relationship between each treatment element and a disease name has been learned by the attention mechanism, each treatment element can be proposed by the additionally trained large language model.

Usually, a disease name and a treatment pattern are often in a simple 1:1 correspondence. However, in a case where the additionally trained large language model is utilized, not only the relationship between the disease name and each treatment element but also the relationship between individual symptoms, physical findings, and test findings are obtained by the attention mechanism. Therefore, it is possible to propose a treatment plan individualized to the situation of each case.

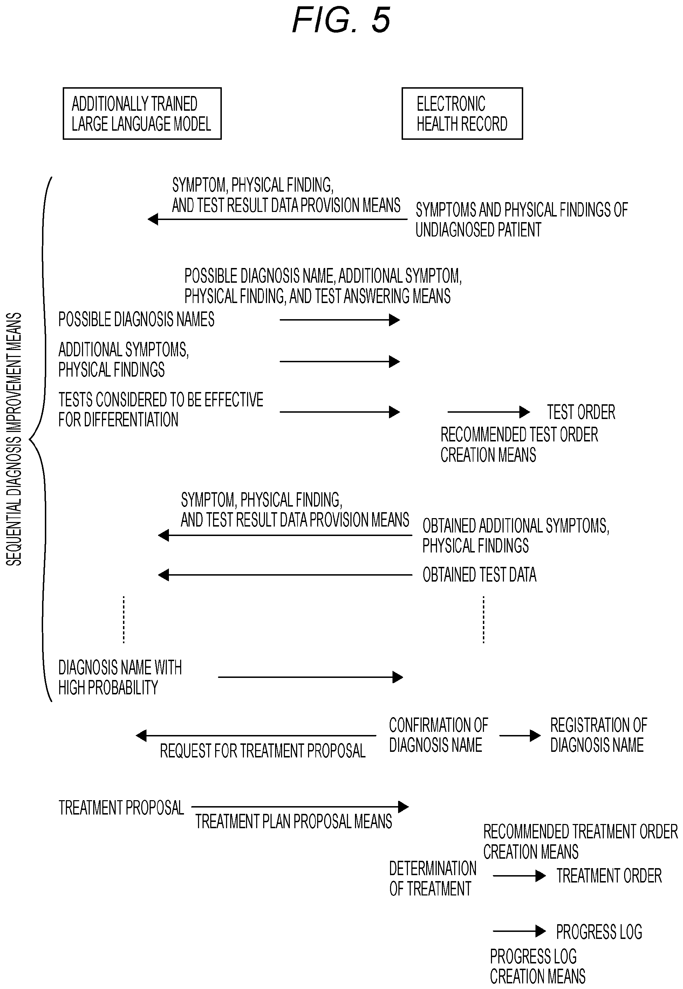

Fig. 5 is an explanatory diagram for creating a diagnosis and treatment plan while receiving a proposal using a reinforcement-learned large language model.

For an undiagnosed patient, a hearing of symptoms and physical findings are obtained and input into an electronic health record.

The hearing of symptoms and the physical findings are input into an additionally trained large language model.

At this time, information extraction using tag information from an electronic health record according to Patent Literature 7: JP 7145367 B2 may also be useful.

From the additionally trained large language model, proposals are made for additional symptoms, physical findings, and tests useful to cite possible diagnosis names and probabilities of the diagnosis names, and diagnostic accuracy.

A doctor inputs the proposed additional symptoms and physical findings, and then selects necessary tests from the suggested tests to create a test order in the electronic health record.

Once additional symptoms, physical findings, and test results are obtained, they are presented to the additionally trained large language model. From the additionally trained large language model, proposals are made again for additional symptoms, physical findings, and tests useful to cite possible diagnosis names and probabilities of the diagnosis names, and diagnostic accuracy.

If a sufficiently high probability is obtained for the possible diagnosis name and probability of the diagnosis name and the doctor is satisfied with the diagnosis name, the diagnosis name is confirmed and registered in the electronic health record. If the probability is still insufficient and the doctor is not satisfied, the same procedure is repeated until the diagnosis name is confirmed (a sequential diagnosis improvement means).

When the diagnosis name is confirmed, the doctor receives a proposal for treatment such as administration medicine, injection, rehabilitation, and surgery which are considered to be appropriate from the additionally trained large language model, selects treatment which is considered to be appropriate, and creates a treatment order in the electronic health record.

Here, the diagnosis name, the individual tests, and the treatment order proposed by the additionally trained large language model are merely proposals, and the final adoption is entrusted to the doctor. This is because the doctor is finally responsible if a problem such as a medical accident occurs.

Here, if there is a progress log creation means for recording the progress logs of the sequential diagnosis improvement means and the treatment plan proposal means, it is possible to verify on what basis the examination and tests were proceeded and the diagnosis was obtained and how the treatment plan was made, and it is possible to provide the "explainability" which conventional AIs lack, which has been a problem.

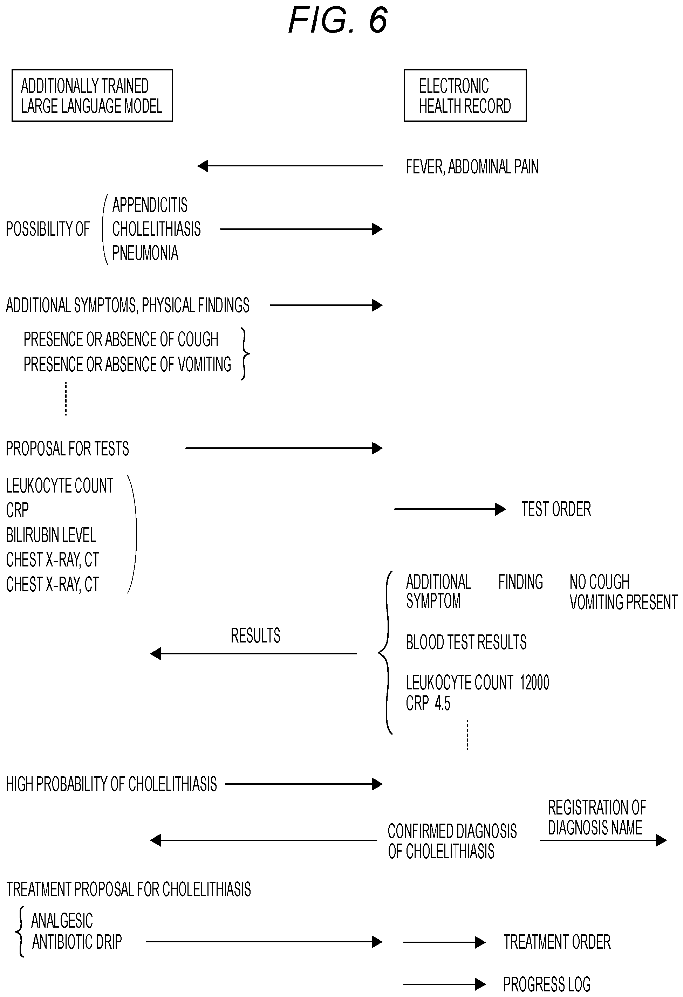

Fig. 6 illustrates an example of the diagnosis and treatment process in Fig. 5.

An undiagnosed patient visited the hospital with fever and abdominal pain. Since a large number of disease names are conceivable for fever and abdominal pain alone, confirmation of the presence or absence of additional symptoms and physical findings such as the presence or absence of cough and vomiting is proposed.

In addition, tests such as a blood test, X-ray, and CT are recommended.

A test order is issued for a test deemed necessary by the doctor. The obtained additional symptoms, physical findings, and test results are returned to the additionally trained large language model, and the additionally trained large language model responds that the diagnosis probability of cholelithiasis is high. When the doctor determines that the diagnosis is confirmed at this time point, the doctor registers the diagnosis name in the electronic health record, and requests the additionally trained large language model for a treatment proposal for cholelithiasis.

If the doctor determines that the proposal for an analgesic or antibiotic drip is appropriate, the doctor issues the treatment order to the electronic health record. Finally, a progress log is created.

In a medical examination by the doctor, first, a medical interview is performed to grasp the symptoms of the patient, the presence or absence of a trigger for causing the symptoms, the time course, and the like, and then a physical finding is obtained. A blood test, a radiation test, and the like for differentiation are ordered to give an approximate indication of a disease name and confirm the disease name, or when a plurality of disease names are suspected. The test result is viewed. If necessary, additional symptoms and physical findings are confirmed, and additional test orders are issued. These are repeated as necessary until the diagnosis of the disease name is assured.

When the possibility of a specific disease name becomes sufficiently high, the disease is registered in the electronic health record as a confirmed disease, and a treatment order necessary for the disease is issued.

In the above-mentioned "giving an approximate indication of a disease name", cases having similar patterns of symptoms and physical findings are often recalled from a large number of cases experienced in the past. However, in this state, the diagnosis is personal and experience varies depending on the doctor, so that it may be difficult to improve the accuracy rate by objective diagnosis. Furthermore, the knowledge of the best doctors would be lost if they die. As in the present invention, continuous accumulation of permanent knowledge is expected to continuously improve diagnosis accuracy.

In order to proceed with diagnosis based on objective criteria, it is necessary to collect a large amount of case data for which diagnosis has been confirmed.

The collection method is optional, but practically, it is a summary at the time of discharge or an outpatient summary at the time of outpatient final examination.

Information extraction using tag information from an electronic health record according to Patent Literature 7: JP 7145367 B2 may also be useful.

The probability of a disease name at the time when there is no prior information at all, for example, an unknown patient enters an examination room at the first visit is a ratio of the frequency of each disease name (prior probability).

Here, when information of symptoms or physical findings is received, the probability of a disease name having the symptoms or physical findings (posterior probability) increases, and the probability of a disease name not having the symptoms or findings decreases. When a plurality of disease names remain as candidates, it is necessary to determine which disease name is correct diagnosis by combining additional symptoms, physical findings, and test results (differential diagnosis).

Here, it goes without saying that effective bases for differential diagnosis are symptoms, physical findings, and tests having a large difference in positive/negative frequency between diseases. By taking symptoms and physical findings having a large positive/negative frequency difference between diseases, performing tests, and providing the results to the additionally trained large language model, the posterior probability for each disease name greatly changes, and it is possible to approach the confirmed diagnosis.

In consideration of the difficulty level and the test cost, the symptoms, physical findings, and tests to be performed next may proceed. This operation may be repeated until the probability of the disease name becomes sufficiently high (a sequential diagnosis improvement means).

Patent Literature 5: JP 6792750 B2 discloses a diagnosis support system that collects a large number of diagnosis names, symptoms, physical findings, and test results (prior probability distribution), and narrows down diagnosis names by a set operation of undiagnosed patient's symptoms, physical findings, and test results (posterior probability).

In addition, Patent Literature 6: JP 7284970 B2 discloses a technique of recording a large amount of records and performing high-speed retrieval called a key value store.

However, there have been problems in that a procedure at the time of inquiry is complicated, adjustment of variation in notation when collecting a large amount of data is complicated, calculation resources required for recording and referring to a large amount of data become enormous, and considerable cost is required.

In the large language model, it is possible to make an inquiry in daily language, and complexity of the inquiry is greatly reduced.

In the attention mechanism adopted in a large language model, the relationship between document elements is learned through estimation learning of masked document elements, and also an additionally trained large language model is created by learning of a similar relationship for the data provided by a reinforcement learning data provision means for diagnosis. Using this mechanism, it is possible to calculate the posterior probability for each disease name after the observed symptoms, physical findings, and test results of the undiagnosed patient are obtained.

Since a huge language system has already been constructed, even recording and processing of a large amount of case data for which diagnosis has been confirmed can be performed at a relatively low cost by utilizing the language model.

Although an embodiment has been described above, the specific configuration of the present invention is not limited to the above embodiment, and design changes and the like without departing from the gist of the invention are included in the present invention.

For example, although medical care has been described as an example, a similar system can be used in nursing care. Although ChatGPT and the like have been exemplified as the LLM, other systems can be similarly used, and a system newly developed in the future is also included in the present invention. In a normal large language model, a query sentence called a prompt is often input to obtain an answer from the large language model. In the present invention, such operation may be performed, or the prompt/answer may be executed via an API between an electronic health record and a large language model.

## Claims

1. A diagnosis and prescription support system comprising:
in an electronic health record system using a large language model in combination,
(1) an additional learning data provision means for diagnosis for performing additional learning by an attention mechanism for obtaining a correlation between an individual symptom, physical finding, test finding, or the like and a disease name of a diagnosed case which is a teacher label, and a correlation between an individual symptom, physical finding, test finding, and the like, as an attention weight of each correlation by using diagnosis name, symptom, physical finding, and test result data of the diagnosed case extracted from an electronic health record, to obtain an additionally trained large language model (a means for creating additionally trained large language model using diagnosed case data);
(2) a "symptom, physical finding, and test result data provision means" that provides symptom, physical finding, and test result data in an electronic health record obtained for an undiagnosed case to the additionally trained large language model;
(3) a "possible diagnosis name, additional symptom, physical finding, and test answering means" that answers, by the additionally trained large language model, at least one of a list of possible diagnosis names and probabilities of the diagnosis names, and a recommended additional symptom, physical finding, and test by calculating probabilities of respective disease names at an intermediate elapsed time point at which the symptom, finding, test result, and the like are obtained, using attention weights of symptom, physical finding, and test result data groups obtained by the symptom, physical finding, and test result data provision means, and a context vector obtained by integrating the attention weights; and
(4) a sequential diagnosis improvement means for repeating the "symptom, physical finding, and test result data provision means" and the "possible diagnosis name, additional symptom, physical finding, and test answering means".

2. The diagnosis and prescription support system according to claim 1, further comprising a recommended test order creation means for creating a test order recommended for an electronic health record by the "possible diagnosis name, additional symptom, physical finding, and test answering means".

3. The diagnosis and prescription support system according to claim 1 or 2, wherein the additional learning provision means for diagnosis includes treatment items such as treatment including administration medicine, injection, rehabilitation, and surgery as well as "symptom, physical finding, and test finding" for proceeding with diagnosis of a diagnosed case.

4. The diagnosis and prescription support system according to claim 1 or 2, wherein the additionally trained large language model includes a treatment plan proposal means for proposing a treatment plan of treatment including administration medicine, injection, rehabilitation, and surgery for a confirmed diagnosis obtained by the sequential diagnosis improvement means.

5. The diagnosis and prescription support system according to claim 4, further comprising a recommended treatment order creation means for creating, for an electronic health record, an individual treatment order such as treatment including administration medicine, injection, rehabilitation, and surgery proposed by the treatment plan proposal means.

6. The diagnosis and prescription support system according to claim 4, further comprising a progress log creation means for recording progress logs of the sequential diagnosis improvement means and the treatment plan proposal means.
